(19) Europäisches Patentamt
European Patent Office
Office européen des brevets

(11) Publication number: **0 012 824**
**B1**

(12) # EUROPEAN PATENT SPECIFICATION

(45) Date of publication of patent specification: **24.03.82**

(51) Int. Cl.³: **C 07 D 311/72**

(21) Application number: **79104413.4**

(22) Date of filing: **09.11.79**

(54) **Process for the manufacture of alpha-tocopherol and novel intermediate in this process.**

(30) Priority: **22.12.78 US 972573**

(43) Date of publication of application:
**09.07.80 Bulletin 80/14**

(45) Publication of the grant of the patent:
**24.03.82 Bulletin 82/12**

(84) Designated Contracting States:
**AT BE CH DE FR GB IT NL**

(56) References cited:
**INDUSTRIE CHIMIQUE BELGE, T35, No. 1, 1970, pages 13—26,**
**B. STALLA-BOURDILLON: "Mises au point sur les synthèses de la vitamine E (α-tocophérol)"**

(73) Proprietor: **F. HOFFMANN-LA ROCHE & CO. Aktiengesellschaft**
**CH-4002 Basel (CH)**

(72) Inventor: **Fitton, Peter**
**49 Colfax Drive**
**Pequannock, N.J. (US)**
Inventor: **Propper, Ronald**
**39-11 Kramer Place**
**Fair Lawn, N.J. (US)**

(74) Representative: **Cottong, Norbert A. et al,**
**124 Grenzacherstrasse P.O. Box 601**
**CH-4002 Basel (CH)**

**EP 0 012 824 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the manufacture of alpha-tocopherol and novel intermediate in this process

The present invention is concerned with a novel process for the manufacture of α-tocopherol which has the formula

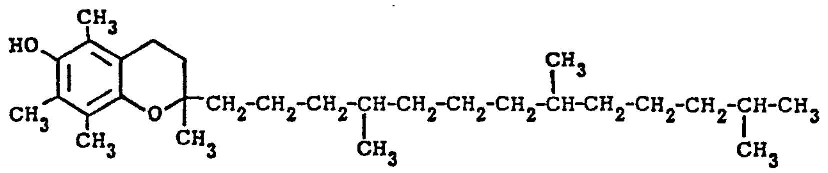

I

This process is characterized in that trimethyl hydroquinone which has the formula

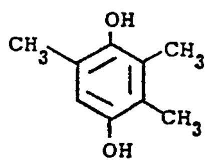

II

is reacted with a phytyl derivative of the general formula

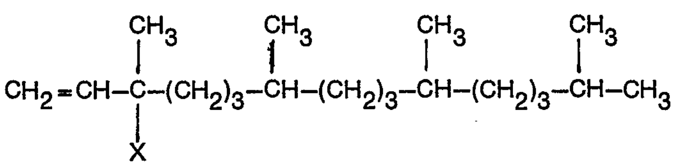

III–A

or

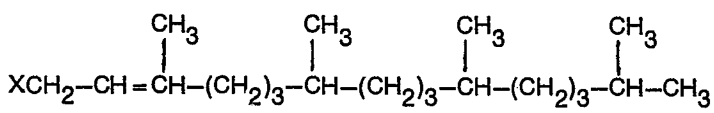

III–B

wherein X is a leaving group,
in the presence of trifluoroacetic acid or trifluoroacetic acid anhydride.

In the past, 2,5,7,8-tetramethyl-2-(4,8,12-trimethyltridecyl)-6-chromanol (α-tocopherol) has already been produced by condensing trimethylhydroquinone with a phytyl derivative. This condensation is generally carried out in the presence of a condensation agent such as a Lewis acid or a hydrohalic acid, (U.S. Patent specifications No. 3,789,086; No. 2,723,278 and No. 2,411,968) at high temperatures, i.e. above 70°C. In these catalytic condensation reactions, the reaction mixture is generally kept saturated with anhydrous hydrogen chloride during the reaction. The anhydrous hydrogen chloride which is generated from concentrated hydrochloric acid is pumped through the reaction mixture and recondensed.

The above process, however, suffers from the disadvantage that it must be carried out at very high temperatures. At very high temperatures, the use of the mentioned condensing agents and hydrogen chloride provides a very corrosive mixture. Furthermore, the use of high temperatures is by itself expensive and energy consuming.

In order to avoid the use of high temperatures, it has subsequently been found that by pretreating the isophytol with amine or ammonia such as disclosed in Derwent No. 65113Y or by utilizing a mixed ether-halohydrocarbon solvent mixture such as disclosed in Derwent No. 65663Y, lower temperatures can be utilized. However, none of these processes avoid the necessity of utilizing acid catalysts as mentioned above and anhydrous hydrogen chloride. A brief summary of prior art processes is given in B. Stalla-Bourdillon, "Industrie Chimique Belge, T35, No. 1, 1970, pp. 13—25.

In utilizing hydrogen chloride, the spent hydrogen chloride is recycled after purification. In order to do this, a considerable amount of the equipment is required solely for processing hydrogen chloride. Also, another disadvantage of this process is that the quality of the α-tocopherol produced presents a purification problem. The impurities in α-tocopherol are difficult to remove since the crude α-tocopherol, under the conditions of fractional distillation, starts to decompose. Therefore, in order to purify the crude α-tocopherol, it must be converted to α-tocopherol acetate by reaction with acetic anhydride. The acetate which is fractionally distilled is hydrolyzed back to α-tocopherol. Even after this extensive purification, the purity of α-tocopherol is only between 90—96%, with major losses in yield since many of the impurities are compounds that are closely related to α-tocopherol. In utilizing this procedure, it has been found that many of the impurities have the same molecular weight and almost

the same boiling point as $\alpha$-tocopherol and are thus practically impossible to remove by standard purification techniques.

All these drawbacks have now been overcome with the novel process as mentioned above.

In accordance with the present invention, it has been unexpectedly found that when a compound of formula II is reacted with a compound of formula III—A or III—B in the presence of trifluoroacetic acid or trifluoroacetic acid anhydride, the compound of formula I is produced at low temperatures, i.e. room temperatures, without the need for utilizing strong acids and/or Lewis acid condensing agents. This is extremely advantageous since condensation occurs at room temperature rather than at temperatures of 110°C, thereby reducing the energy costs and costs of using high temperature equipment. Furthermore, there is no need to use Lewis acids such as zinc chloride or ferric chloride as catalysts in the condensation reaction, thereby avoiding possible pollution problems. The elimination of the use of concentrated hydrochloric acid and the resulting anhydrous hydrogen chloride in the reaction provides considerable advantages. Besides substantially eliminating the corrosion problem, the need to generate anhydrous hydrogen chloride is also eliminated. Furthermore, in the process of this invention, there is no need to purify and recycle the hydrogen chloride.

It has also been found that the process of this invention produces $\alpha$-tocopherol in higher yields and with higher purities than is obtained by the prior processes. In fact, purities as high as 99.5 to 100% $\alpha$-tocopherol are obtained due to the elimination of those impurities which have the same molecular weight and almost the same boiling point as $\alpha$-tocopherol. By utilizing the process of the present invention, there is no need to convert the crude $\alpha$-tocopherol to $\alpha$-tocopherol acetate for purification, thereby also eliminating a costly step utilized in prior processes.

As used through the specification and claims, the term "halogen" denotes all four halogens, i.e. bromine, chlorine, fluorine and iodine, with bromine and chlorine being especially preferred.

The term "lower alkyl" denotes saturated aliphatic hydrocarbon groups containing 1 to 7 carbon atoms, such as methyl, ethyl, propyl, isopropyl and isobutyl.

The term "lower alkoxy" denotes lower alkoxy groups containing from 1 to 7 carbon atoms such as methoxy, ethoxy, propyloxy, n-butoxy and isobutoxy.

The term "lower alkanoyl" denotes lower alkanoyl groups containing from 1 to 7 carbon atoms, preferably from 2 to 7 carbon atoms such as acetyl or propionyl.

The term "aryl" as used herein, denotes monocyclic aromatic hydrocarbons such as phenyl and polycyclic aromatic hydrocarbons such as naphthyl, which may be unsubstituted or substituted in one or more positions with a lower alkyl or nitro group.

In accordance with this invention, the substituent X in the compounds of formulae III—A and III—B can be any conventional leaving group. Among the preferred leaving groups are included hydroxy, halides, lower alkylsulfonyloxy, arylsulfonyloxy, lower alkoxy, and lower alkanoyloxy. Among the preferred halides are chlorine and bromine. The preferred lower alkylsulfonyloxy leaving group is mesyloxy. The preferred arylsulfonyloxy leaving group is tosyloxy. The preferred lower alkanoyloxy group is acetoxy. Among the preferred lower alkoxy leaving groups are methoxy, ethoxy, n-butoxy, isobutoxy and tert.-butoxy.

In accordance with the present invention, the reaction of the compound of formula II with a compound of formula III—A or III—B is carried out in the presence of either trifluoroacetic acid or trifluoroacetic acid anhydride. In carrying out this reaction, good yields are obtained utilizing room temperature, i.e temperatures of from 20° to 30°C. While it is generally preferred to carry out this reaction at room temperature, lower or higher temperatures can be utilized. In general, temperatures of from −5°C to 70°C are utilized.

The trifluoroacetic acid or trifluoroacetic acid anhydride can be utilized as the solvent medium. Therefore, it is not necessary to utilize additional solvents. However, if desired, other solvents can be utilized in the reaction medium. Among the other solvents which can be utilized are included such conventional solvents as toluene, heptane, methylene chloride, acetic acid, diethyl ether and aromatic hydrocarbon solvents such as benzene and xylene. The reaction of this invention can be carried out without the necessity of utilizing a Lewis acid, concentrated hydrochloric acid or other mineral acids. These materials may however, if desired, be present, in the reaction medium. In view of the dilatorious effect of these materials, little if any advantage is seen in their use, especially in the use of large quantities of mineral acids required by the prior art for carrying out this reaction.

In the reaction of a compound of formula III—A or III—B with a compound of the formula II to produce a compound of the formula I, the compound of the following formula

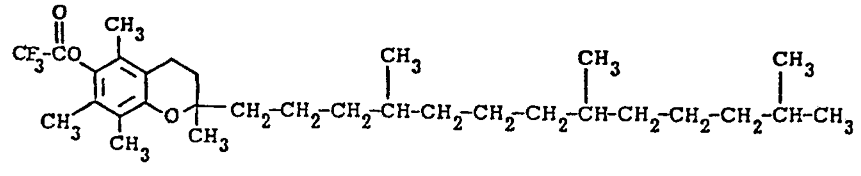

IV

can, if desired, be formed as an intermediate. This intermediate is formed in a mixture with $\alpha$-tocopherol. This intermediate in the reaction mixture can be converted directly to $\alpha$-tocopherol by

treating the reaction mixture with a base. Therefore, there is no need to isolate the compound of formula IV from the reaction medium. On the other hand, if desired, the compound of formula IV can be isolated in a mixture with the compound of formula I from the reaction medium. The compound of formula IV can be separated from the reaction medium by any conventional method of separation. Among the preferred methods of separating the compound of formula IV from the compound of formula I is by chromatography. Any conventional method of chromatography can be utilized to effect the separation.

The compound of formula IV can also be converted to the compound of formula I by basic hydrolysis. Any conventional method of basic hydrolysis can be utilized to effect this conversion. Among the preferred methods is by treating the compound of formula IV with an aqueous inorganic base. Among the aqueous bases are the strong bases such as alkali metal hydroxides e.g. sodium hydroxide, potassium hydroxide, lithium hydroxide. In carrying out this hydrolysis, it is generally preferred to utilize room temperature, i.e. from 20° to 30°C. However, if desired, higher or lower temperatures can be utilized, i.e. from 0° to 100°C.

The compound of formula IV is novel and forms also part of the present invention.

The invention is further illustrated by the following examples. In the examples, the term "glc" denotes gas liquid chromatography.

Example 1

255 g isophytol (98%) were added, dropwise, over a period of three hours to a vigorously stirred solution of 145.0 g trimethylhydroquinone in 750 ml trifluoroacetic acid at room temperature under nitrogen. When the addition was complete, the mixture was stirred for a further 30 minutes and then concentrated on a rotary evaporator (35°C/30 mmHg) to give an oil. This oil was dissolved in 1 liter hexane and the solution was washed with 2 x 500 ml methanol:water (1:1, v:v) and 500 ml 1N aqueous sodium bicarbonate solution. 200 ml of a 1% by weight solution of potassium hydroxide in methanol were added and this mixture was stirred at room temperature for 30 minutes, 200 ml cold 10% by weight aqueous hydrochloric acid were then aded. The organic layer was separated, washed with 500 ml 1N aqueous sodium bicarbonate solution and 500 ml water, and then concentrated on a rotary evaporator (50°C/40 mm) to leave 353.0 g of a brown oil. This oil was flash-distilled and the following fractions were collected.

| Fraction | bp (°C) | Pressure mmHg | wt. (g) | Area % of α-tocopherol by glc |
|---|---|---|---|---|
| 1 | 137–234 | 0.05 | 12.3 | 47.6 |
| 2 | 234–259 | 0.05 | 9.1 | 91.0 |
| 3 | 259–278 | 0.05 | 312.1 | 99.7 |

Example 2

25.5 g isophytol (98%) were added dropwise over three hours to a mixture of 14.5 g trimethylhydroquinone, 65 ml trifluoroacetic acid and 10 ml water. The product was diluted with 200 ml hexane, washed with 3 x 100 ml methanol-water (1:1, v:v) and 100 ml saturated aqueous sodium bicarbonate solution. 100 ml of a 1% by weight solution of potassium hydroxide in methanol were added and the mixture was stirred at room temperature for 30 minutes. Then 100 ml 2N aqueous hydrochloric acid were added, the organic phase was separated, washed with 100 ml saturated aqueous sodium bicarbonate solution and 100 ml methanol:water (1:1, v/v). The hexane was removed by concentrating on a rotary evaporator and the resulting oil was flash-distilled to give 29.6 g α-tocopherol; bp 220°C/0.1 mmHg. Purity by area % glc was 100%.

Example 3

25.5 g isophytol (98%) were added dropwise over a period of two hours to a mixture of 14.5 g trimethylhydroquinone in 150 ml trifluoroacetic acid at –5°C.

The resulting product was then refluxed for 22 hours under nitrogen, after which the excess trifluoroacetic acid was removed by concentration on a rotary evaporator (35°C/30 mmHg). The resulting oil was dissolved in 100 ml hexane, the solution was washed with 3 x 100 ml methanol-water (1:1, v/v) and dried over $Na_2SO_4$. Removal of the hexane left an oil that on flash distillation (205—215°C/0.2 mmHg) gave 36.8 g of an oil, which by glc analysis was shown to be a mixture of α-tocopherol (6.7% by weight) and α-tocopherol trifluoroacetate (88.5%).

The above mixture as the oil after removal of hexane was again diluted with 200 ml hexane, washed with 3 x 100 ml methanol-water (1:1, v:v) and 100 ml saturated aqueous sodium bicarbonate

solution. 100 ml of a 1% by weight solution of potassium hydroxide in methanol was added and the mixture was stirred at room temperature for 30 minutes. Then 100 ml of 2N aqueous hydrochloric acid were added, the organic phase was separated and washed with 100 ml saturated aqueous sodium bicarbonate solution and 100 ml methanol-water (1:1, v:v). The hexane was removed by concentrating on a rotary evaporator and the resulting oil was flash-distilled to give $\alpha$-tocopherol; bp 220°C/0.1 mmHg. Purity by area % glc was 100%.

Example 4

255 g (0.84 moles) isophytol (98%) were added dropwise over a period of one hour to a vigorously stirred solution of 130.8 g (0.86 moles) trimethylhydroquinone in 750 ml trifluoroacetic acid at room temperature under nitrogen. The mixture was stirred for a further 30 minutes and then concentrated on a rotary evaporator (35°—40°/30 mmHg) to leave a brown oil. This oil was dissolved in 1000 ml hexane and the solution was then washed with 2 × 500 ml methanol:water (1:1, v/v) and 500 ml 1N saturated aqueous sodium bicarbonate solution. 200 ml of a 1% solution of potassium hydroxide in methanol were added and the mixture was stirred at room temperature for 30 minutes. 200 ml cold 10% aqueous hydrochloric acid were added, the organic layer was separated and washed with 500 ml 1N aqueous sodium bicarbonate solution and 500 ml water, then concentration on a rotary evaporator, leaving 376.0 g of a brown oil. Glc analysis of this oil showed that it contained 88.5% by weight $\alpha$-tocopherol (by use of an internal standard). This oil was flash distilled and the following fractions were collected:

| Fraction | bp (°C) | Pressure (mmHg) | Wt (g) | % Tocopherol by glc: Area % | % Tocopherol by glc: Wt % (internal standard) |
|---|---|---|---|---|---|
| 1 | 87–240 | 0.2 | 29.0 | 62.7 | - |
| 2 | 240–246 | 0.2 | 12.43 | 99.4 | 99.0 |
| 3 | 246–260 | 0.12 | 289.91 | 99.9 | 99.9 |
| 4 | 260–280 | 0.12 | 12.99 | 99.8 | 89.9 |

## Claims

1. Process for the manufacture of $\alpha$-tocopherol of the formula

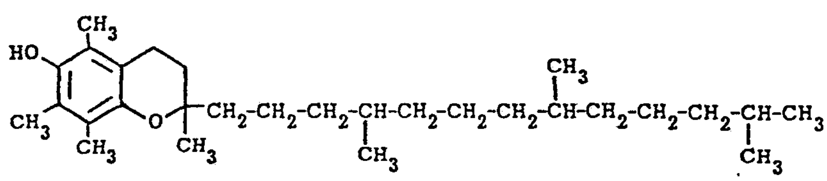

I

by reacting the compound of the formula

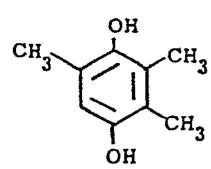

II

with a phytyl derivative of the general formula

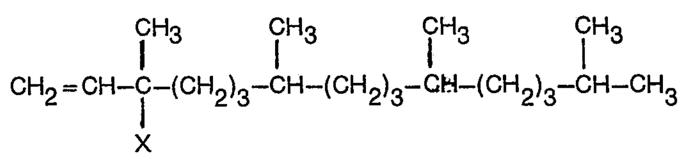

III–A

or

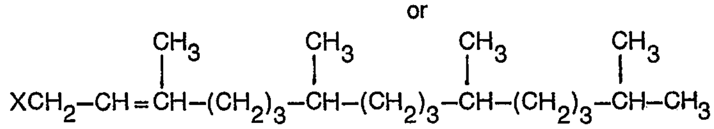

III–B

wherein X is a leaving group characterized in that the reaction is carried out in the presence of trifluoroacetic acid or trifluoroacetic acid anhydride.

2. Process according to claim 1, characterized in that the compound of formula II is reacted with a compound of formula III—A or III—B, wherein X is arylsulfonyloxy, ($C_1$—$C_7$) alkylsulfonyloxy, hydroxy, halogen, ($C_1$—$C_7$) alkoxy and ($C_1$—$C_7$) alkanoyloxy.

3. Process according to claim 1 or 2, characterized in that the compound of formula II is reacted with a compound of formula III—A or III—B, wherein X is hydroxy, toxyloxy, acetoxy or tert. butoxy.

4. Process according to claim 1, 2 or 3, characterized in that the compound of formula II is reacted with isophytol.

5. Process according to any one of claims 1 to 4, characterized in that the reaction is carried out in the presence of trifluoroacetic acid.

6. Process according to any one of claims 1 to 5, characterized in that the reaction is carried out at a temperature from −5°C to 70°C.

7. Process according to claim 6, characterized in that the reaction is carried out at a temperature from 20°C to 30°C.

8. The compound of the formula

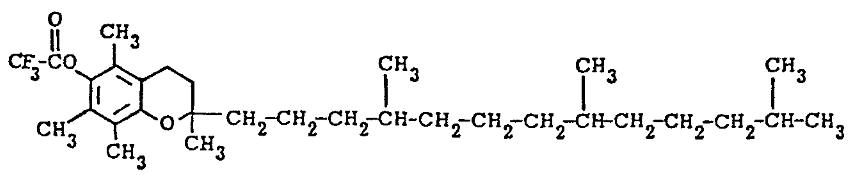

## Patentansprüche

1. Verfahren zur Herstellung von $\alpha$-Tocopherol der Formel

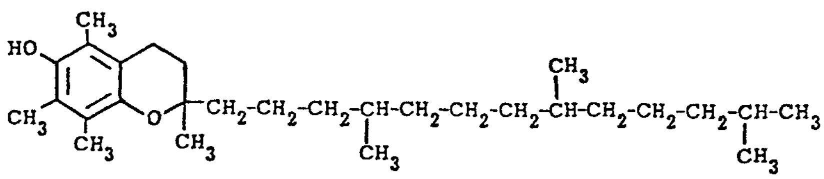

I

durch Umsetzung der Verbindung der Formel

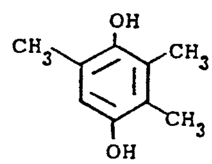

II

mit einem Phytylderivat der allgemeinen Formel

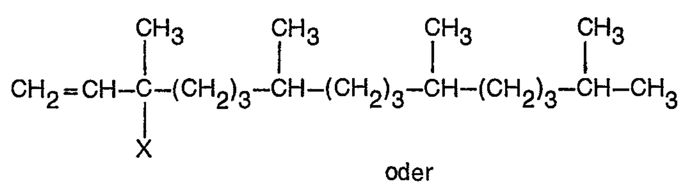

III–A

oder

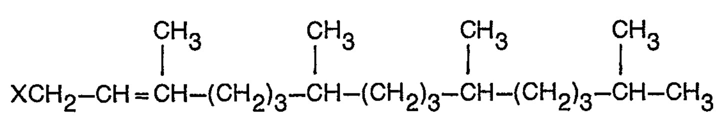

III–B

worin X eine austretende Gruppe bedeutet, dadurch gekennzeichnet, dass man die Umsetzung in Gegenwart von Trifluoressigsäure oder Trifluoressigsäureanhydrid durchführt.

2. Verfahren gemäss Anspruch 1, dadurch gekennzeichnet, dass man die Verbindung der Formel II mit einer Verbindung der Formel III—A oder III—B, worin X Arylsulfonyloxy, ($C_1$—$C_7$)-Alkylsulfonyloxy, Hydroxy, Halogen, ($C_1$—$C_7$)-Alkoxy oder ($C_1$—$C_7$)-Alkanoyloxy bedeutet, umsetzt.

3. Verfahren gemäss Anspruch 1 oder 2, dadurch gekennzeichnet, dass man die Verbindung der Formel II mit einer Verbindung der Formel III—A oder III—B, worin X Hydroxy, Tosyloxy, Acetoxy oder tert. Butoxy bedeutet, umsetzt.

4. Verfahren gemäss Anspruch 1, 2 oder 3, dadurch gekennzeichnet, dass man die Verbindung der Formel II mit Isophytol umsetzt.

5. Verfahren gemäss einem der Ansprüche 1 bis 4, dadurch gekennzeichnet, dass man die Umsetzung in Gegenwart von Trifluoressigsäure durchführt.

6. Verfahren gemäss einem der Ansprüche 1 bis 5, dadurch gekennzeichnet, dass man die Umsetzung bei einer Temperatur von −5°C bis 70°C durchführt.

7. Verfahren gemäss Anspruch 6, dadurch gekennzeichnet, dass man die Umsetzung bei einer Temperatur von 20°C bis 30°C durchführt.

8. Die Verbindung der Formel

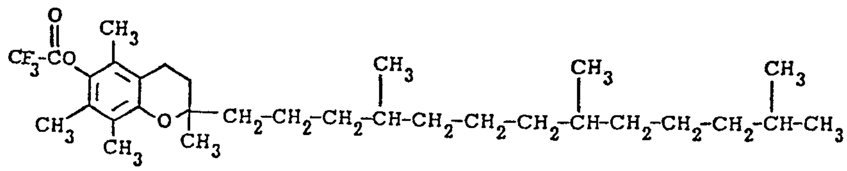

**Revendications**

1. Procédé pour la préparation d'alpha-tocophérol de la formule:

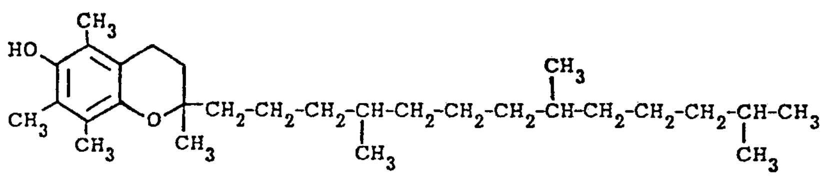

I

en faisant réagir le composé de la formule:

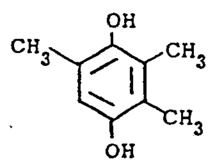

II

avec un dérivé de phytyle de la formule générale:

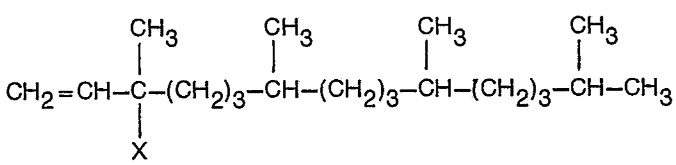

III–A

ou

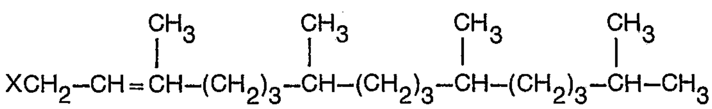

III–B

où X est un groupe qui part, caractérisé en ce que la réaction est conduite en présence d'acide trifluoroacétique ou d'anhydride d'acide trifluoroacétique.

2. Procédé selon la revendication 1, caractérisé en ce que le composé de formule II est mis à réagir avec un composé de formule III—A ou III—B, dans laquelle X est un groupe arylsulfonyloxy, alcoyl (en $C_1$—$C_7$) sulfonyloxy, hydroxy, un halogène ou un groupe alcoxy en $C_1$—$C_7$ ou alcanoyloxy en $C_1$—$C_7$.

3. Procédé selon l'une des revendications 1 et 2, caractérisé en ce que le composé de formule II est mis à réagir avec un composé de formule III—A ou III—B, dans laquelle X est un groupe hydroxy, tosyloxy, acétoxy ou tert-butoxy.

4. Procédé selon l'une des revendications 1 à 3, caractérisé en ce que le composé de formule II est mis à réagir avec l'isophytol.

5. Procédé selon l'une des revendications 1 à 4, caractérisé en ce que la réaction est conduite en présence d'acide trifluoro-acétique.

6. Procédé selon l'une des revendications 1 à 5, caractérisé en ce que la réaction est conduite à une température comprise entre −5°C et 70°C.

7. Procédé selon la revendication 6, caractérisé en ce que la réaction est conduite à une température comprise entre 20°C et 30°C.

8. Le composé de la formule